(19) Europäisches Patentamt
European Patent Office
Office européen des brevets

(11) Veröffentlichungsnummer : **0 465 410 B1**

# (12) EUROPÄISCHE PATENTSCHRIFT

(45) Veröffentlichungstag der Patentschrift : **01.06.94 Patentblatt 94/22**

(51) Int. Cl.$^5$ : **C09B 5/62,** C08K 5/3437

(21) Anmeldenummer : **91810466.2**

(22) Anmeldetag : **18.06.91**

## (54) Perylentetracarbonsäurediimide mit langkettigen Alkanoylaminoresten.

(30) Priorität : **27.06.90 CH 2143/90**

(43) Veröffentlichungstag der Anmeldung : **08.01.92 Patentblatt 92/02**

(45) Bekanntmachung des Hinweises auf die Patenterteilung : **01.06.94 Patentblatt 94/22**

(84) Benannte Vertragsstaaten : **DE FR GB IT**

(56) Entgegenhaltungen :
**EP-A- 0 006 122**
**EP-A- 0 033 079**
**EP-A- 0 283 436**
**BE-A- 562 588**
**DE-B- 2 832 761**
**US-A- 2 668 815**

(73) Patentinhaber : **CIBA-GEIGY AG**
**Klybeckstrasse 141**
**CH-4002 Basel (CH)**

(72) Erfinder : **Hari, Stefan, Dr.**
**Ringstrasse 5**
**CH-4153 Reinach (CH)**

EP 0 465 410 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft Perylentetracarbonsäurediimide mit an beiden N-Atomen über Aromaten gebundenen langkettigen Alkanoylaminoresten und ihre Verwendung zum Färben von hochmolekularem organischem Material.

In US Patent 2 668 815 sind Perylentetracarbonsäurediimide mit an beiden N-Atomen über Phenylen gebundenen kurzkettigen oder cyclischen Alkanoylaminoresten als Küpenfarbstoffe beschrieben.

Aus dem US Patent 4 238 386 sind N,N'-Dialkylperylen-3,4,9,10-tetracarbonsäurediimide mit langkettigen Alkylresten zum Färben von Polyolefinen bekannt. Diese Produkte genügen allerdings einerseits wegen ihrer Ausblühtendenz, und andererseits bezüglich ihrer Nassechtheiten nicht immer den heutigen Anforderungen der Technik.

Das US Patent 4 262 851 beschreibt Perylen-3,4,9,10-tetracarbonsäurediimide, die an beiden N-Atomen durch niedere Alkanoylaminogruppen substituiert sind und ihre Verwendung zum Färben von Lacken, Anstrichmitteln und Kunststoffen. Auch diese Pigmente genügen nicht immer den inzwischen für bestimmte Anwendungen stets höher gesetzten Erfordernissen.

Aus der EP-PS 283 436 ist bekannt, dass man bei Verwendung von Perylentetracarbonsäurediimiden mit an beide N-Atome gebundenen, Carboxy- oder Carbamoylgruppen enthaltenden langkettigen aliphatischen Resten gefärbte Polyolefine mit bereits besseren Beständigkeiten erhalten kann.

Nun sind aber neue Perylentetracarbonsäurediimide, an deren beide N-Atome Aromate mit langkettigen Alkanoylaminoresten gebunden sind, gefunden worden, die beim Färben von hochmolekularem organischem Material überraschenderweise noch beständigere Ausfärbungen ergeben.

Die vorliegende Erfindung betrifft demnach Verbindungen der Formeln

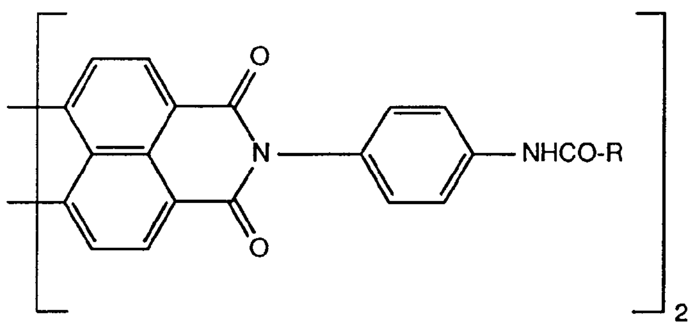

(I),

worin R $C_{12}$-$C_{18}$-Alkyl ist, und

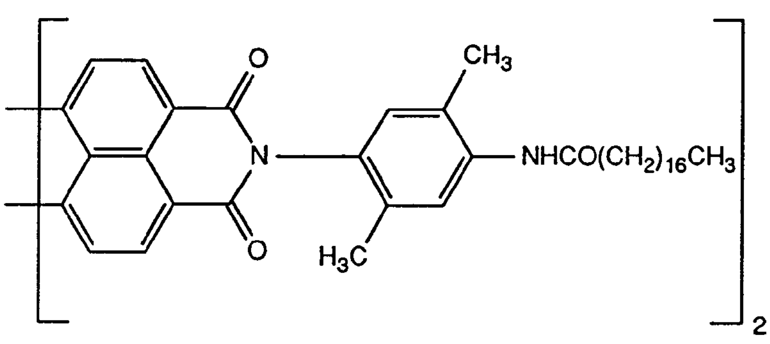

(Ia).

R bedeutet als $C_{12}$-$C_{18}$-Alkyl z.B. Dodecyl, Tridecyl, Tetradecyl, Pentadecyl, Hexadecyl oder Octadecyl.

Die Verbindungen der Formel I können nach allgemein bekannten Methoden hergestellt werden. Zweckmässig werden sie in zwei Syntheseschritten, ausgehend von Perylen-3,4,9,10-tetracarbonsäure oder deren Anhydrid, z.B. durch eine erste Kondensation mit einer Aminogruppe von Phenylendiamin und dann eine zweite Kondensation des erhaltenen Diaminodiimids mit einem Carbonsäurechlorid der Formel

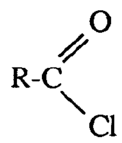

(II),

worin R die oben angegebene Bedeutung hat, gemäss folgendem Reaktionsschema erhalten: Perylen-3,4,9,10-tetracarbonsäure bzw. -anhydrid + mindestens

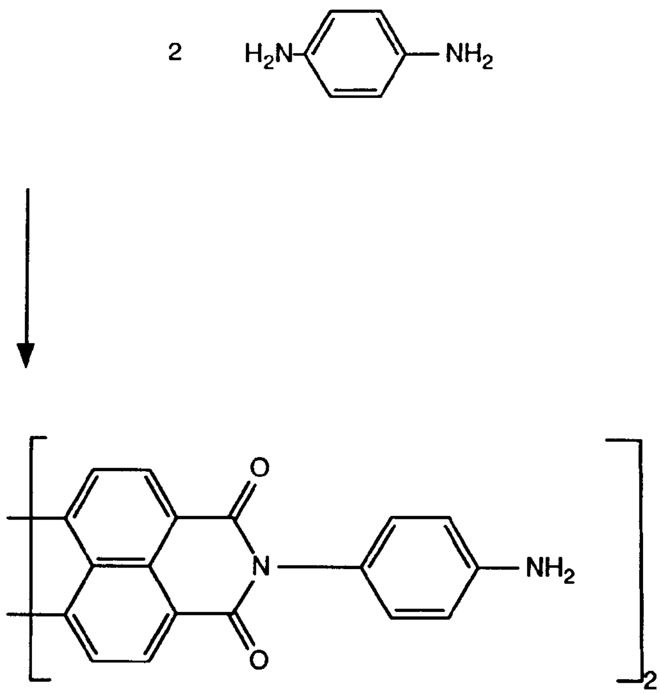

+ mindestens

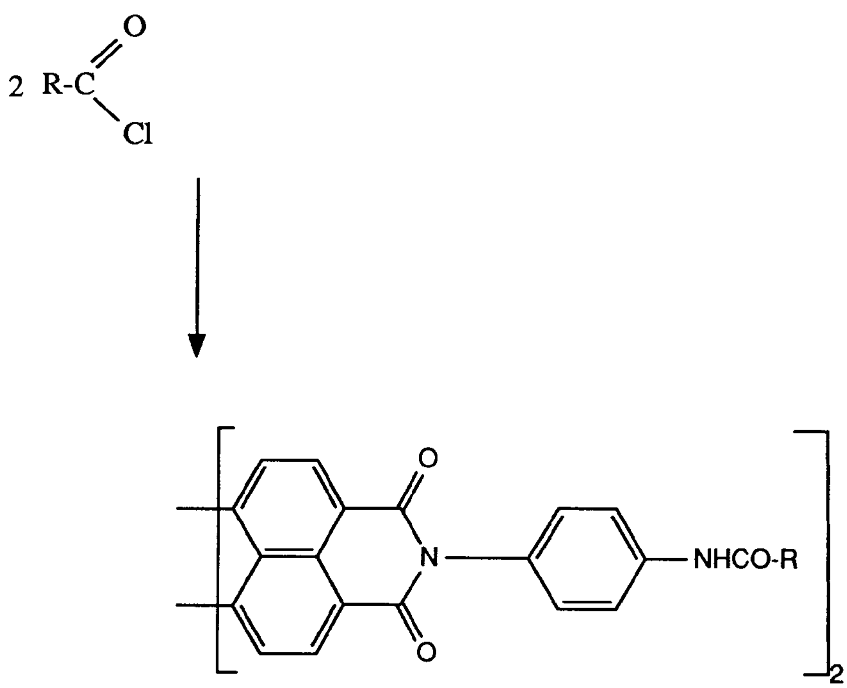

Die Carbonsäurechloride der Formel II lassen sich zweckmässig aus den entsprechenden bekannten und ebenfalls grösstenteils im Handel erhältlichen Carbonsäuren, z.B. durch Umsetzung mit Thionylchlorid, nach bekannten Methoden herstellen. Es kann aber auch die freie Säure direkt oder ein Niederalkylester davon zur Umsetzung gebracht werden.

Die Kondensationsreaktionen können nach allgemein üblichen Methoden, beispielsweise in Gegenwart organischer Lösungsmittel oder bei Verwendung eines der entsprechenden Reaktionspartner als Lösungsmittel, bei Normal- oder Ueberdruck, mit oder ohne Katalysator, durchgeführt werden.

Als Lösungsmittel für die erste Kondensationsreaktion zur Bildung der entsprechenden Perylendiimide ausgehend von Perylen-3,4,9,10-tetracarbonsäure oder deren Dianhydrid sind z.B. Wasser, Dimethylformamid, N-Methylpyrrolidon, Chinolin, Glykole, wie Ethylen- oder Propylenglykol, Alkohole, wie Methanol, Ethanol, n-Propylalkohol, Isopropylalkohol, n-Butylalkohol und dessen Isomere oder Diacetonalkohol, sowie aromatische Kohlenwasserstoffe, wie Nitrobenzol, Chlorbenzol, Dichlor- und Trichlorbenzole, Toluol und Xylole geeignet.

Als Lösungsmittel für die zweite Kondensationsreaktion seien beispielsweise Cyclohexan, aromatische Kohlenwasserstoffe, wie Nitrobenzol, Chlorbenzol, Dichlor- und Trichlorbenzole, Toluol, Xylole, Cumol und tert.-Butylbenzol erwähnt.

Verwendet man bei den Kondensationsreaktionen einen Reaktionspartner direkt als Lösungsmittel (im Ueberschuss), so wird das Reaktionsmedium nach vollständiger Amidbildung vorzugsweise mit einem der oben erwähnten organischen Lösungsmittel verdünnt, bis die im Ueberschuss vorliegende Komponente in gelöstem Zustand vorliegt, dann wird die verbleibende Suspension abfiltriert. Aus dem Filtrat lässt sich die überschüssige Komponente durch Abdampfen des Lösungsmittels zurückgewinnen.

Eine weitere Möglichkeit, die Verbindungen der Formel I herzustellen, besteht darin, Perylen-3,4,9,10-tetracarbonsäuredianhydrid in Analogie zu den obenerwähnten allgemein üblichen Methoden mit einem Amin der Formel

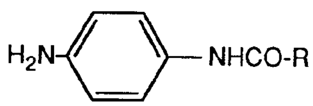

(III)

im Molverhältnis 1:2 zu kondensieren.

Die Amine der Formel III sind bekannt oder können nach üblichen Methoden hergestellt werden.

Die erhaltenen erfindungsgemässen Perylendiimide werden nach üblichen Methoden isoliert und getrocknet.

Die erfindungsgemässen Perylendiimide eignen sich ausgezeichnet als Pigmente zum Färben von hochmolekularem organischem Material.

Hochmolekulare organische Materialien, die mit den erfindungsgemässen Pigmenten gefärbt bzw. pigmentiert werden können, sind z.B. Celluloseether und -ester, wie Ethylcellulose, Nitrocellulose, Celluloseacetat oder Cellulosebutyrat, natürliche Harze oder Kunstharze, wie Polymerisations- oder Kondensationsharze, wie Aminoplaste, insbesondere Harnstoff- und Melamin-Formaldehydharze, Alkydharze, Phenoplaste, Polycarbonate, Polyolefine, Polystyrol, Polyvinylchlorid, Polyamide, Polyurethane, Polyester, ABS, Polyphenylenoxide, Gummi, Casein, Silikon und Silikonharze, einzeln oder in Mischungen.

Die erwähnten hochmolekularen organischen Verbindungen können einzeln oder in Gemischen als plastische Massen, Schmelzen oder in Form von Spinnlösungen, Lacken, Anstrichstoffen oder Druckfarben vorliegen. Je nach Verwendungszweck erweist es sich als vorteilhaft, die erfindungsgemässen Pigmente als Toner oder in Form eines Präparates einzusetzen.

Besonders geeignet sind die erfindungsgemässen Pigmente zum Einfärben von Polyvinylchlorid und insbesondere Polyolefinen, wie Polyethylen und Polypropylen, in der Masse sowie zum Pigmentieren von Lacken und Anstrichstoffen, insbesondere Automobillacken und darunter bevorzugt Metalleffektlacke.

Bezogen auf das zu pigmentierende hochmolekulare organische Material kann man die erfindungsgemässen Pigmente in einer Menge von 0,01 bis 30 Gew.%, vorzugsweise von 0,1 bis 10 Gew. %, einsetzen.

Die Pigmentierung der hochmolekularen organischen Substanzen mit den erfindungsgemässen Pigmenten erfolgt beispielsweise derart, dass man das Pigment, gegebenenfalls in Form von Masterbatches, diesen Substraten unter Verwendung von Walzwerken, Misch- oder Mahlapparaten zumischt. Das pigmentierte Material wird hierauf nach an sich bekannten Verfahren, wie Kalandrieren, Pressen, Strangpressen, Streichen, Giessen oder Spritzgiessen, in die gewünschte endgültige Form gebracht. Oft ist es erwünscht, zur Herstellung von nicht starren Formlingen oder zur Verringerung ihrer Sprödigkeit den hochmolekularen Verbindungen vor der Verformung sogenannte Weichmacher einzuverleiben. Als solche können z.B. Ester der Phosphorsäure, Phthalsäure oder Sebacinsäure dienen. Die Weichmacher können vor oder nach der Einverleibung des erfindungsgemässen Pigmentes in die Polymeren eingearbeitet werden. Zwecks Erzielung verschiedener Farbtöne ist es ferner möglich, den hochmolekularen organischen Stoffen neben dem erfindungsgemässen Pigment noch Füllstoffe bzw. andere farbgebende Bestandteile, wie Weiss-, Bunt- oder Schwarzpigmente, in beliebigen Mengen zuzufügen.

Zum Pigmentieren von Lacken, Anstrichstoffen und Druckfarben werden die hochmolekularen organischen Materialien und die erfindungsgemässen Pigmente gegebenenfalls zusammen mit Zusatzstoffen, wie Füllmitteln, anderen Pigmenten, Siccativen oder Weichmachern, in einem gemeinsamen organischen Lösungsmittel oder Lösungsmittelgemisch fein dispergiert bzw. gelöst. Man kann dabei so verfahren, dass man die einzelnen Komponenten für sich oder auch mehrere gemeinsam dispergiert bzw. löst, und erst hierauf alle Komponenten zusammenbringt.

In Färbungen, beispielsweise von Polyvinylchlorid oder Polyolefinen, zeichnen sich die erfindungsgemässen Pigmente durch gute allgemeine Pigmenteigenschaften, wie gute Dispergierbarkeit, hohe Farbstärke und Reinheit, sowie ausgezeichnete Migrations-, Hitze-, Licht- und Wetterbeständigkeit aus. Sie eignen sich auch besonders gut für die Einfärbung von Lacken für Metalleffektlackierungen. Zudem zeigen sie eine ausgezeichnete Säurebeständigkeit.

Die nachfolgenden Beispiele erläutern die Erfindung.

Beispiel 1: Eine Mischung aus 14,25 g N,N'-Bis-4-aminophenyl-perylentetracarbonsäure diimid und 27,75 g Stearinsäurechlorid in 500 ml o-Dichlorbenzol wird mit 10 ml Pyridin versetzt und auf 140°C erhitzt. Die dunkelrote Suspension wird 6 Stunden bei dieser Temperatur gerührt und danach auf 80°C abgekühlt und filtriert. Das Produkt wird nacheinander mit 100 ml o-Dichlorbenzol, 1000 ml heissem Alkohol und 1000 ml heissem Wasser gewaschen und bei 60°C im Vakuumtrockenschrank getrocknet. Man erhält 25,55 g (92,4 % der Theorie) eines roten Pigments der Formel

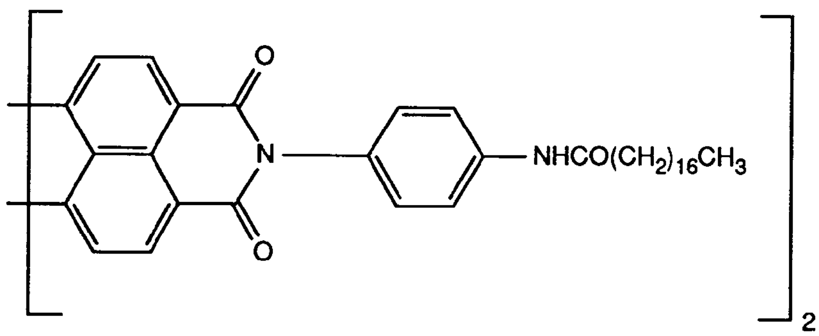

in Pulverform, das z.B. Polyethylen, Polyvinylchlorid und Polyamid in farbstarken roten Tönen von ausgezeichneter Migrations-, Hitze- und Lichtbeständigkeit färbt.

Analyse:

Ber. (in %): C 78,23 H 8,02 N 5,07

Gef. (in %): C 77,86 H 7,84 N 4,84

Beispiele 2-4: Weitere Pigmente können nach dem in Beispiel 1 beschriebenen Verfahren hergestellt werden, wenn man jeweils das in Kolonne I der nachfolgenden Tabelle angegebene Diamin mit dem in Kolonne II angegebenen Säurechlorid im Molverhältnis 1:2 acyliert. Kolonne m charakterisiert die Nuance der mit den erhaltenen Pigmenten gemäss nachfolgendem Beispiel 5 eingefärbten PVC-Folie.

| Bsp. | I | II | III |
|---|---|---|---|
| 2 | N,N'-Bis-4-aminophenyl-perylen-tetracarbonsäurediimid | Myristinsäurechlorid | rot |
| 3 | N,N'-Bis-4-aminophenyl-perylen-tetracarbonsäurediimid | Palmitoylchlorid | rot |
| 4 | N,N'-Bis-2,5-dimethyl-4-amino-phenyl-perylentetracarbonsäure-diimid | Stearoylchlorid | orange |

Beispiel 5: 40 mg des gemäss Beispiel 1 erhaltenen Perylendiimidpigmentes werden mit 7,3 ml Dioctylphthalat und 13,3 g eines stabilisierten Polyvinylchlorids vom Typ ®LONZA E-722 in einem Becherglas mit einem Glasstab gut vermischt. Das erhaltene Gemisch wird dann auf einem Walzenstuhl während 5 Minuten bei 160°C zu einer dünnen Folie verarbeitet. Die so erzeugte PVC-Folie weist eine farbstarke rote Färbung hoher Reinheit und sehr guter Lichtbeständigkeit auf. Die Dispergierbarkeit des Pigmentes ist ausgezeichnet.

Beispiel 6: Eine Mischung von 1,0 g des nach Beispiel 1 erhaltenen Perylendiimidpigmentes, 1,0 g Antioxidans ®IRGANOX 1010 (CIBA-GEIGY AG) und 1000 g Polyethylen HD Granulat (®VESTOLEN A60-16, HUELS) wird während 15 Minuten in einer 3 l-Glasflasche auf einer Rollbank vorgemischt. Danach wird die Mischung zweimal durch einen Einwellenextruder extrudiert. Das so erhaltene Granulat wird dann auf einer Spritzgussmaschine (®Allround Aarburg 200) bei 250°C mit einer Verweilzeit von 5 Minuten zu Platten verspritzt. Die so erhaltenen Platten weisen gleichmässig farbstarke rote Färbungen hoher Reinheit und ausgezeichneter Lichtbeständigkeit auf.

Beispiel 7: 1000 g Polypropylengranulat (®DAPLEN PT-55, Chemie LINZ) und 1,0 g des nach Beispiel 1 erhaltenen Perylendiimidpigmentes werden in einer 3 l-Flasche während 15 Minuten auf dem Rollbock gemischt. Danach wird die Mischung zweimal durch einen Einwellenextruder extrudiert und anschliessend granuliert. Das so erhaltene Granulat wird bei 280-285°C nach dem Schmelzspinnverfahren versponnen. Die so gefärbten roten Fasern weisen eine sehr gute Lichtbeständigkeit und ausgezeichnete textile Echtheiten, wie Reib- und Nassechtheiten gegenüber Wasch- und Lösungsmitteln, auf. Die Hitzebeständigkeit des Pigments während des Spinnprozesses bei 285°C ist ausgezeichnet.

Beispiel 8: Verfährt man analog wie in Beispiel 6 beschrieben, verwendet aber neben dem Buntpigment 10 g Titandioxid ®KRONOS RN-57-P (KRONOS Titan GmbH), so erhält man rote Pressplatten mit ebensoguten Hitzebeständigkeiten. Die zwischen 200 und 280°C gespritzten Pressplatten weisen nach dem Erkalten keine Farbabweichungen auf.

Beispiel 9:

Eine Mischung von
130 g Steatitkugeln (∅ = 8 mm),
45,5 g eines thermohärtenden Acryllacks, bestehend aus
- 41,3 g Acrylharz ®VIACRYL VC 373,60 % (VIANOVA Kunstharz AG),
- 16,3 g Melaminharz ®MAPRENAL TTX, 55 % (HOECHST AG),
- 32,8 g Xylol,
- 4,6 g Ethylglykolacetat,
- 2,0 g Butylacetat und
- 1,0 g ®Silikonöl A, 1 % in Xylol (BAYER AG), und

2,5 g des nach Beispiel 1 erhaltenen Perylendiimids

werden in einer 200 ml Glasflasche mit "Twist-off"-Verschluss während 72 Stunden auf einem Rollgestell dispergiert. Nach Abtrennen der Steatitkugeln werden 8,0 g der so dispergierten Volltonmischung, 0,6 g Aluminium-Paste ®ALCOA, (60-65 % Al-Gehalt, Aluminium Corp. of America), 1,0 g Methylethylketon und 18,4 g des oben erwähnten thermohärtenden Acryllacks gut vermischt, das so erhaltene Gemisch wird auf Aluminiumbleche gespritzt und anschliessend während 30 Minuten bei 130°C eingebrannt. Man erhält sehr farbstarke rote Metalleffektlackierungen mit ausgezeichneten Beständigkeiten.

**Patentansprüche**

1. Verbindungen der Formeln

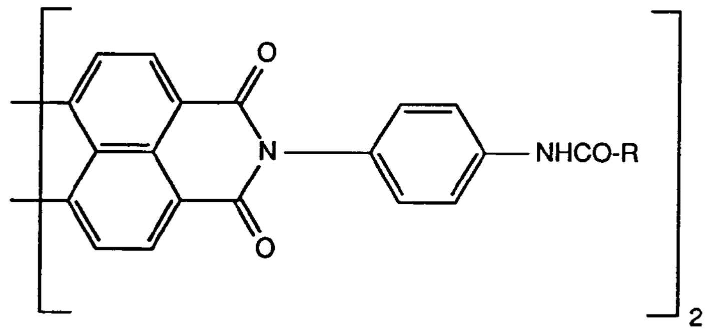

(I),

worin R $C_{12}$-$C_{18}$-Alkyl ist, und

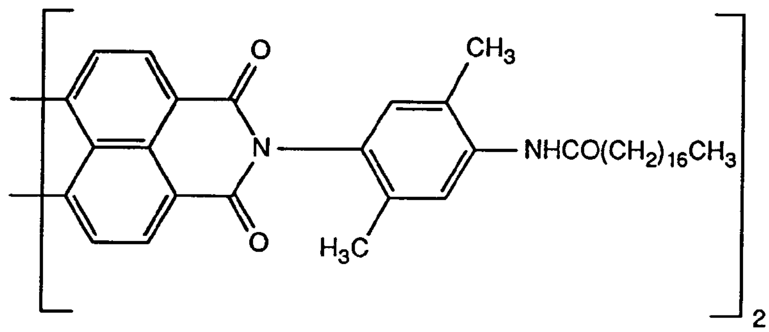

(Ia).

2. Hochmolekulares organisches Material enthaltend als Pigment eine Verbindung der Formel I gemäss Anspruch 1.

3. Hochmolekulares organisches Material gemäss Anspruch 2, dadurch gekennzeichnet, dass es ein Polyolefin ist.

4. Hochmolekulares organisches Material gemäss Anspruch 2, dadurch gekennzeichnet, dass es ein Lack ist.

**Claims**

1. A compound of formula

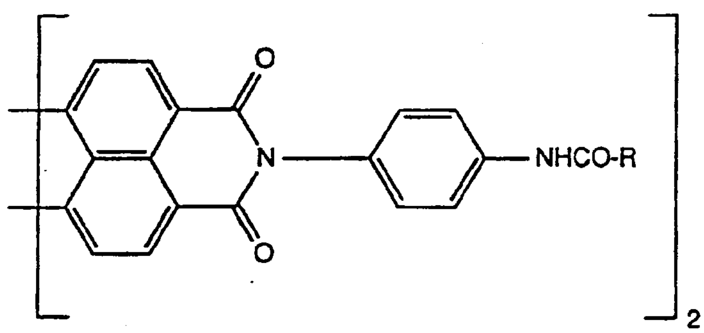

(I),

wherein R is $C_{12}$-$C_{18}$alkyl, or

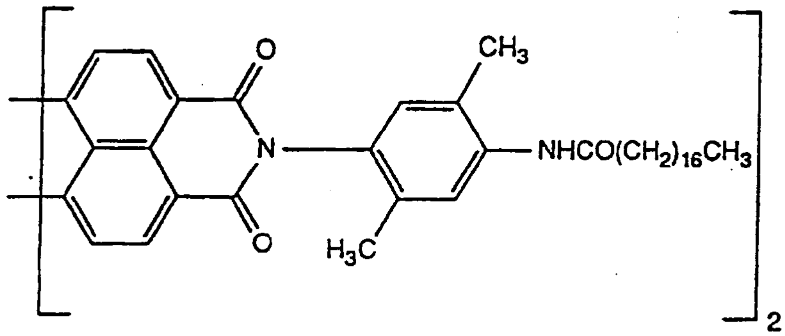

(Ia).

2. Organic material of high molecular weight pigmented with a compound of formula I according to claim 1.

3. organic material of high molecular weight according to claim 2, which is a polyolefin.

4. organic material of high molecular weight according to claim 2, which is a paint.

**Revendications**

1. Composés de formules :

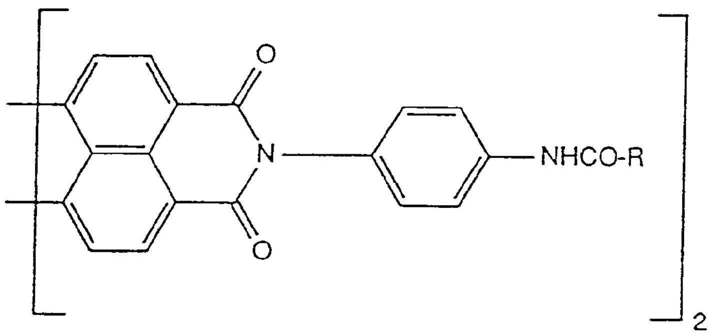

(I)

R étant un alkyle en $C_{12}$-$C_{18}$, et

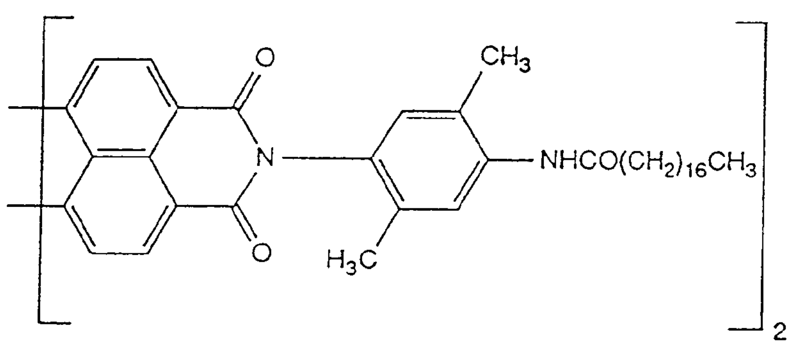

(Ia)

2. Matières organiques macromoléculaires contenant comme pigment un composé de formule I selon la revendication 1.

3. Matière organique macromoléculaire selon la revendication 2, qui est une polyoléfine.

4. Matière organique macromoléculaire selon la revendication 2, en une laque ou peinture.